# EUROPÄISCHE PATENTSCHRIFT

(11) **EP 1 016 382 B1**
(45) Veröffentlichungstag und Bekanntmachung des Hinweises auf die Patenterteilung: **10.05.2006**
(21) Anmeldenummer: 99124270.2
(22) Anmeldetag: 04.12.1999
(51) Int. Cl.: A61B 17/74

(54) **Schenkelhalsschraube**
Femoral neck screw
Vis pour le col du fémur

(30) Priorität: 28.12.1998 DE 29823113 U
(43) Veröffentlichungstag der Anmeldung: 05.07.2000
(73) Patentinhaber: Stryker Trauma GmbH, 24232 Schönkirchen (DE)
(72) Erfinder: Harder, Hans Erich, 24253 Probsteierhagen (DE); Karich, Bernhard, 02627 Kubschütz, OT Purschwitz (DE)
(74) Vertreter: Hauck, Graalfs, Wehnert, Döring, Siemons

(56) Entgegenhaltungen:
- EP-A- 0 257 118
- EP-A- 0 551 588
- GB-A- 2 209 947
- US-A- 3 996 931
- US-A- 5 112 333

## Beschreibung

Die Erfindung bezieht sich auf eine Schenkelhalsschraube nach dem Oberbegriff des Anspruchs 1.

Aus EP 0 257 118 ist ein Osteosynthesehilfsmittel bekanntgeworden, das aus einem Verriegelungsnagel und einer Schenkelhalsschraube besteht. Der Verriegelungsnagel wird von proximal in den Femur eingetrieben. Der Verriegelungsnagel weist im proximalen Abschnitt eine schräge Durchbohrung auf, deren Achse annähernd zur Achse des Schenkelhalses ausgerichtet ist. Durch diese Bohrung wird eine Schenkelhalsschraube hindurchgeführt. Sie weist einen Gewindeteil auf, der zum Beispiel selbstschneidend ist und der bis in den Kopf des Femurs vorgetrieben wird. Vom proximalen Ende wird in eine Bohrung des Verriegelungsnagels eine Feststellschraube eingeführt, die mit ihrem inneren Ende mit in Umfangsrichtung beabstandeten achsparallelen Nuten im Schaft der Schenkelhalsschraube zusammenwirkt, um die Schenkelhalsschraube gegen Rotation zu sichern, jedoch ein Gleiten in Achsrichtung zuzulassen. Ein derartiges Osteosynthesehilfsmittel dient vorwiegend zur Versorgung von trochanteren und subtrochanteren Frakturen, aber auch zur Versorgung von Frakturen des Schenkelhalses oder von Frakturen im Kopfbereich.

Mit Hilfe einer Kompressionsschraube, die in den distalen Bereich der Schenkelhalsschraube eingeschraubt wird und die mit dem Verriegelungsnagel zusammenwirkt, kann auch eine Kompression ausgeübt werden. Dieser Anwendungsfall tritt bei Brüchen im Kopf- und Halsbereich auf.

Zur Anbringung des Verriegelungsnagels und der Halsschraube ist ein Zielinstrumentarium erforderlich, um von außen die Bohrungen im Verriegelungsnagel aufzufinden. Hierfür gibt es die verschiedensten Vorschläge. Die meisten beinhalten ein Instrument, das auf das proximale Ende des Verriegelungsnagels aufgesetzt wird und einen Bügel aufweist, der sich im Abstand parallel zum Femur erstreckt, wenn mit dem Verriegelungsnagel verbunden.

Bei der Verwendung des beschriebenen Osteosynthesehilfsmittels kann es bei der Versorgung pertrochanterer Femurfrakturen mit kurzem Kopf-Hals-Fragment oder bei erheblicher Osteoporose zu Komplikationen im Bereich der Schenkelhalsschraube kommen. Die Schenkelhalsschraube kann bei reduzierter Knochensubstanz oder kranialer Fehllage ausbrechen. Ferner besteht die Gefahr der sekundären Rotation des Kopf-Hals-Fragments bei exzentrischer Lage de Halsschraube. Es ist ferner bekannt, statt einer Schenkelhalsschraube eine Klinge zu verwenden, welche durch eine Öffnung im Verriegelungsnagel hindurchgeführt wird. Im Hinblick auf die Rotationsstabilität ist eine derartige Klinge günstiger. Das Einbringen der Klinge ist jedoch weitaus problematischer. Außerdem läßt sich mit Hilfe einer Klinge eine Frakturkompression nicht erzielen.

Der Erfindung liegt die Aufgabe zugrunde, eine Schenkelhalsschraube zu schaffen, bei der die lastaufnehmende Fläche im Gewindeteil der Schenkelhalsschraube vergrößert wird.

Diese Aufgabe wird durch die Merkmale des Anspruchs 1 gelöst.

Bei der erfindungsgemäßen Schenkelhalsschraube sind ausgehend vom distalen Ende auf gegenüberliegenden Außenseiten achsparallele Nuten geformt, von denen mindestens eine sich bis in den Gewindeteil hineinerstreckt. Ferner ist eine gabelförmige Klinge vorgesehen, deren Klingenschenkel von den Nuten aufgenommen sind. Der Querschnitt der Klingenschenkel entspricht vorzugsweise dem der Nuten, wobei jedoch ein geringes Spiel gelassen ist. Am distalen Ende sind die Klingenschenkel über einen geeigneten Verbindungsabschnitt miteinander verbunden, der seinerseits in geeigneter Weise mit dem distalen Ende der Halsschraube verbunden wird. Vorzugsweise ist der Verbindungsabschnitt ringzylindrisch und in seinem Außendurchmesser annähernd gleich dem Außendurchmesser des Schaftes der Schenkelhalsschraube. Er kann mit Hilfe einer Schraube, die in ein Innengewinde am distalen Ende der Schenkelhalsschraube eingedreht wird, befestigt werden. Vorzugsweise hat der ringzylindrische Abschnitt seinerseits ein Innengewinde für die Verbindung mit einem geeigneten Einschlaginstrument.

Mindestens eine Schenkelhalsklinge erstreckt sich bis in den Bereich des Gewindeteils der Halsschraube oder auch geringfügig darüber hinaus. Vorzugsweise sind jedoch beide Klingenschenkel annähernd gleich lang, so daß beide die lastaufnehmende Fläche im Gewindeteil der Schenkelhalsschraube vergrößern. Dadurch ergibt sich eine effektive Sicherung gegen sekundäre Kopf-Hals-Rotation. Die erfindungsgemäße Schenkelhalsschraube ist besonders vorteilhaft bei der Anwendung bei erheblicher Osteoporose oder exzentrischer Hüftschraubenlage.

Die Nuten bzw. die Klingenschenkel sind vorzugsweise so ausgelegt, daß im Bereich des Schaftes der Halsschraube die Außenseiten der Klingenschenkel annähernd in Höhe der Außenseite des Schaftes liegen. Die Nuten können nach einer anderen Ausgestaltung der Erfindung im Bereich des Gewindeteils flacher verlaufen, wobei eine Rampe zwischen den verschieden tiefen Nutabschnitten vorgesehen werden kann.

Dadurch kommt es zu einer Spreizung der Klingenschenkel und damit zu einer wirksameren Verankerung.

Nach einer anderen Ausgestaltung der Erfindung weisen die Enden der Klingenschenkel außen eine Anschrägung auf. Dadurch wird das Eintreiben erleichtert. Nach einer anderen Ausgestaltung der Erfindung sind die Enden der Klingenschenkel an der Innenseite angeschrägt, wodurch das Einführen in die Nuten erleichtert wird. Diesem Ziel dienen auch optionale Anschrägungen in den Nutwänden im Eintrittsbereich der Klingen sowie im Boden der Nut.

Die Implantation der erfindungsgemäßen Schenkelhalsschraube erfolgt in üblicher Weise. Nach ihrem Eindrehen wird sie in Drehrichtung festgelegt, wie das bei dem eingangs beschriebenen Osteosynthesehilfsmittel der Fall ist, etwa mit Hilfe einer Feststellschraube und achsparallelen Nuten an der Außenseite des Schraubenschaftes. Ggf. kann eine Kompression der Fraktur vorgenommen werden. Anschließend erfolgt das Einschlagen der Klinge, was über einen Bildwandler verfolgt werden kann. Ist die Erstreckung der Klingenschenkel derart, daß sie über das proximale Ende der Halsschraube hinausragen, kann dies am Bildwandler festgestellt werden. Nach dem Entfernen des Einschlagelements kann die Klinge gegen ein Herausgleiten mit Hilfe einer Schraube, die in das beschriebene Innengewinde am proximalen Ende der Schenkelhalsschraube eingedreht wird, gesichert werden.

Die erfindungsgemäße Schenkelhalsschraube ist zu dem herkömmlichen oben beschriebenen Osteosynthesehilfsmittel vollständig kompatibel. Es kann das herkömmliche Zielinstrumentarium verwendet werden. Die dynamische Lagerung der Schenkelhalsschraube kann beibehalten werden. Die Handhabung der erfindungsgemäßen Schenkelhalsschraube ist einfach. Auch das Explantieren bereitet keine Probleme. Die erfindungsgemäße Schenkelhalsschraube kann jedoch wahlweise auch ohne Arretierungsklinge eingesetzt werden.

Der einzige Unterschied, der sich etwa gegenüber den oben beschriebenen herkömmlichen Schenkelhalsschrauben ergibt ist der, daß nur zwei Gleit- und Feststellnuten im Schraubenschaft geformt werden können, weil die die Klinge aufhehmenden Nuten hierfür nicht in Frage kommen.

Die Erfindung wird nachfolgend anhand eines in Zeichnungen dargestellten Ausführungsbeispiels näher erläutert.
- Fig. 1: zeigt schematisch in Seitenansicht eine Schenkelhalsschraube nach der Erfindung.
- Fig. 2: zeigt eine Schraube, die in das distale Ende der Schraube nach Fig. 1 einschraubbar ist.

- Fig. 3: zeigt eine erste Seitenansicht einer Klinge zwecks Verbindung mit der Schraube nach Fig. 1.
- Fig. 4: zeigt die um 90° verdrehte Seitenansicht der Klinge nach Fig. 3.
- Fig. 5: zeigt die Endansicht der Schraube nach Fig. 1 in Richtung Pfeil 5.
- Fig. 6: zeigt die Endansicht der Schraube nach Fig. 1 in Richtung Pfeil 6.
- Fig. 7: zeigt die Endansicht der Klinge nach Fig. 3 in Richtung Pfeil 7.
- Fig. 8: zeigt die Endansicht der Klinge nach Fig. 3 in Richtung Pfeil 8.
- Fig. 9: zeigt die Einzelheit vergrößert des Klingenendes nach Fig. 4.

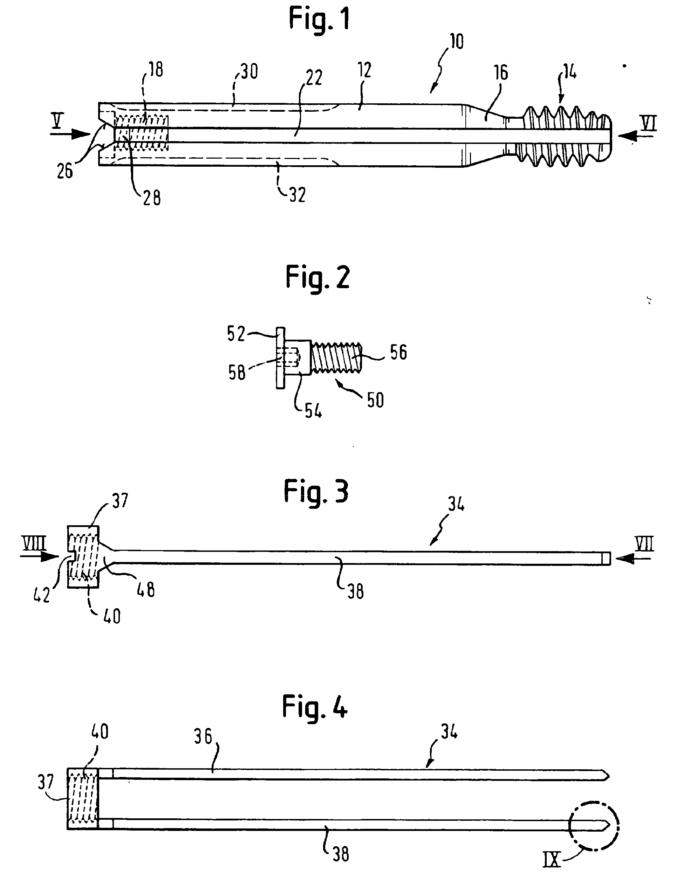

Eine Schenkelhalsschraube 10 nach Fig. 1 weist einen zylindrischen Schaft 12 auf und ein Gewindeteil 14, wobei der Schaft 12 über einen konischen Abschnitt 16 in den Gewindeteil 14 übergeht. Der Spitzendurchmesser des selbstschneidenden Gewindes des Gewindeteils 14 entspricht annähernd dem Außendurchmesser des Schaftes 12. Der Gewindeteil 14 läuft in das proximale Ende der Schraube 10 aus. Am distalen Ende weist der Schaft 12 einen Innengewindeabschnitt 18 auf, der sich fortsetzt in eine Durchbohrung 20. Die Durchbohrung dient für die Aufnahme eines Führungsspießes beim Implantieren der Schenkelhalsschraube.

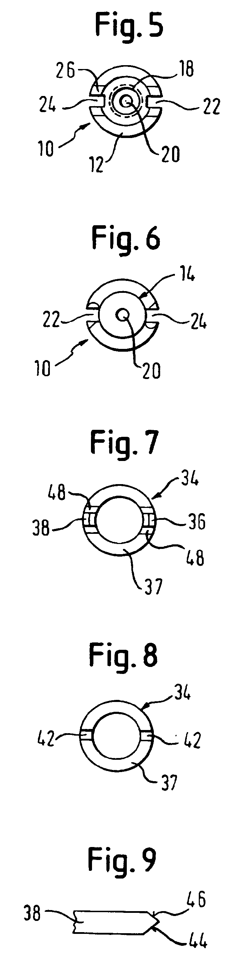

Auf diametral gegenüberliegenden Seiten erstrecken sich Nuten über die gesamte Länge des Nagels 10, wobei in Fig. 1 nur eine Nut 22 zu erkennen ist, während in den Figuren 5 und 6 auch eine Nut 24 zu sehen ist. Die Nuten haben annähernd quadratischen Querschnitt. Die Nutwandung erweitert sich nach distal in einer Schrägung, wie bei 26 in Fig. 1 zu erkennen. Der Nutgrund weist eine Anschrägung 28 auf, d.h. die Nut 22, 24 ist vom distalen Ende her gesehen zunächst tiefer als im restlichen Bereich. Die Nuten 22, 24 können im übrigen im Bereich des Gewindeteils 14 eine etwas geringere Tiefe haben. Dies ist jedoch nicht dargestellt.

Auf diametral gegenüberliegenden Seiten, mithin jeweils um 90° gegenüber den Nuten 22, 24 versetzt, sind Gleitnuten 30, 32 im hinteren bzw. distalen Bereich des Schaftes 12 eingeformt. Sie sind in Fig. 1 gestrichelt angedeutet. Auf ihre Funktion wird weiter unten noch eingegangen.

In den Figuren 3 und 4 bzw. 7 und 8 ist eine Klinge 34 dargestellt. Sie besteht aus zwei parallelen Klingenschenkeln 36, 38, die einteilig mit einem ringzylindrischen Abschnitt 37 geformt sind. Die Klingenschenkel 36, 38 haben quadratischen Querschnitt und sind so bemessen, daß sie annähernd passend von den Nuten 22, 24 aufgenommen sind, wobei dann die Außenseiten der Klingenschenkel 36, 38 annähernd in Höhe der Außenseite des Schaftes 12 liegen. Die Außenseiten der Klingenschenkel 36, 38 können entsprechend zum Radius des Schaftes 12 gerundet sein, erforderlich ist dies jedoch nicht.

Der ringzylindrische Abschnitt 37 hat einen Außendurchmesser, der annähernd dem Außendurchmesser des Schaftes 12 der Schraube 10 entspricht. Er weist außerdem einen Innengewindeabschnitt 40 auf, in den ein Gewindeabschnitt eines nicht gezeigten Einschlaginstruments eingeschraubt werden kann. An dem den Schenkeln 36, 38 gegenüberliegenden Ende weist der ringzylindrische Abschnitt 37 zwei diametrale Einschnitte 42 auf. In diese können entsprechende Vorsprünge des Einschlaginstruments eingreifen, um eine Drehung zwischen dem Einschlaginstrument und der Klinge 34 zu verhindern.

Wie insbesondere aus Fig. 9 hervorgeht, in der das vordere Ende der Klinge 34 dargestellt ist, weisen die Klingen 36, 38 am Ende an der Außenseite eine erste Anschrägung 44 und an der inneren Seite eine Anschrägung 46 auf, die eine Spitze bilden. Die Anschrägung 46 wirkt mit der Anschrägung 28 im Boden der Nuten 22, 24 zusammen, um das Einführen der gabelförmigen Klinge zu erleichtern. Hierzu dienen auch die Anschrägungen 26 in den Nutwandungen, wobei nach dem vollständigen Einführen der Klingenschenkel 36, 38 in die Nuten 22, 24 die dreieckig verbreiterten Abschnitte 48 in den Raum eingreifen, der zwischen zwei Anschrägungen 26 gebildet ist. Dabei liegt die den Klingen zugewandte Stirnseite des ringzylindrischen Abschnitts 37 gegen das distale Ende der Schraube 10 an. Die Spitzen der Klingenschenkel 36, 38 stehen dabei etwas über das proximale Ende der Schenkelhalsschraube 10 über.

Eine Befestigungsschraube 50 gemäß Fig. 2 dient zur Befestigung des ringzylindrischen Abschnitts 37 am distalen Ende der Schraube 10. Die Befestigungsschraube 50 weist einen scheibenartigen Kopf 52 auf von einem Durchmesser, der annähernd dem Außendurchmesser des Schaftes 12 entspricht. Hieran schließt sich ein glatter Abschnitt 54 an, dessen Außendurchmesser annähernd dem Spitzendurchmesser des Innengewindeabschnitts 40 des ringzylindrischen Abschnitts entspricht. Ein Gewindeabschnitt 56 wirkt mit dem Innengewinde 18 des Schaftes 12 zusammen.

Bei der Implantation wird die Schraube 10 zum Beispiel durch die Schrägdurchbohrung eines Verriegelungsnagels hindurchgeführt, nachdem ein entsprechendes Loch im Femur gebohrt wurde. Ferner erfolgt eine Vorbohrung im Hals und Kopf des Femurs. Die Schenkelhalsschraube wird dann in den Hals bzw. Kopf des Femurs eingeschraubt, wie dies an sich bekannt ist. Eine Feststellschraube, die am proximalen Ende des Nagelschaftes angebracht ist, kann mit einer der Nuten 30, 32 zusammenwirken, um nach dem Eindrehen der Schraube 10 eine weitere Drehung zu verhindern. Um auch die axiale Lage der Schraube 10 zu sichern, kann die Feststellschraube angezogen werden, daß sie kraftschlüssig mit einer der Nuten 30, 32 zusammenwirkt und somit auch eine axiale Sicherung für die Schraube 10 darstellt. Das Einschrauben der Schenkelhalssschraube 10 erfolgt mit Hilfe eines nicht gezeigten Führungsspießes, der zuvor eingetrieben worden ist und auf dem die Schraube 10 "aufgefädelt" wird, wobei sich der Spieß durch die zentrale Bohrung 20 hindurcherstreckt.

Anschließend wird die in den Figuren 1 bis 3 und 4 bzw. 7 und 8 dargestellte Klinge eingeschlagen, und zwar mit Hilfe eines Instruments, das mit dem Gewindeabschnitt 40 zusammenwirkt.

Die Klingenschenkel 36, 38 werden in die Nuten 22, 24 eingeführt und vorgetrieben, bis der ringzylindrische Abschnitt 37 gegen das distale Ende der Schraube 10 anschlägt. Die spitzen Enden der Klingenschenkel 36, 38 stehen dabei etwas über das proximale Ende der Schraube 10 über. Danach wird mit Hilfe der Schraube 50 die Klinge 34 am Schaft 12 der Schraube 10 festgelegt. Zur Betätigung weist die Schraube 50 eine Einsenkung 58 auf mit Schraubenflächen (Inbus).

Sobald die Klinge 34 auf die beschriebene Weise befestigt ist, kann die Feststellschraube im Hinblick auf eine der Nuten 30, 32 etwas gelöst werden, so daß die Schraube 10 zwar nach wie vor gegen Drehung gesichert ist, jedoch axial gleiten kann.

## Patentansprüche

1. Schenkelhalsschraube (10), mit einem Schaft (12), einem Außengewindeteil (14) am proximalen Ende und Werkzeugangriffsflächen am distalen Ende, wobei die Schraube so ausgebildet ist, daß sie durch eine Bohrung einer am Femur anbringbaren Stützvorrichtung, vorzugsweise eines Verriegelungsnagels, hindurchgeführt und darin gehalten werden kann, **dadurch gekennzeichnet, daß** ausgehend vom distalen Ende auf gegenüberliegenden Seiten achsparallele Nuten (22, 24) geformt sind, von denen mindestens eine sich bis in den Gewindeteil (14) erstreckt und eine gabelförmige Klinge (34) vorgesehen ist, deren Klingenschenkel (36, 38) von den Nuten (22, 24) aufgenommen sind, während der Verbindungsabschnitt (37) der Schenkel (36, 38) mit dem distalen Ende des Schraubenschaftes (12) verbindbar ist.

2. Schenkelhalsschraube nach Anspruch 1, **dadurch gekennzeichnet, daß** beide Nuten (22, 24) und die Klingenschenkel (36, 38) sich in den Gewindeteil (14) hineinerstrecken.

3. Schenkelhalsschraube nach Anspruch 2, **dadurch gekennzeichnet, daß** die Klingenschenkel (36, 38) sich über das proximale Ende der Schraube (10) hinaus erstrecken.

4. Schenkelhalsschraube nach einem der Ansprüche 1 bis 3, **dadurch gekennzeichnet, daß** die Nuten (22, 24) bzw. die Klingenschenkel (36, 38) im Bereich des Schaftes (12) so bemessen sind, daß die Außenseite der Klingenschenkel (36, 38) annähernd in Höhe der Außenseite des Schaftes (12) liegen.

5. Schenkelhalsschraube nach Anspruch 4, **dadurch gekennzeichnet, daß** die Nuten (22, 24) im Bereich des Gewindeteils (14) flacher sind, gegebenenfalls einen Rampenabschnitt zwischen den Nutabschnitten unterschiedlicher Tiefe aufweisen.

6. Schenkelhalsschraube nach einem der Ansprüche 1 bis 5, **dadurch gekennzeichnet, daß** die Klingenschenkel (36, 38) einteilig mit einem ringzylindrischen Abschnitt (37) geformt sind, dessen Außendurchmesser annähernd dem Außendurchmesser des Schraubenschaftes (12) ist und eine Schraube (50) vorgesehen ist zur Befestigung des ringzylindrischen Abschnitts (37) am distalen Ende des Schraubenschaftes (12).

7. Schenkelhalsschraube nach Anspruch 6, **dadurch gekennzeichnet, daß** der ringzylindrische Abschnitt (37) ein Innengewinde (40) aufweist für die Anbringung an einem Einschlaginstrument.

8. Schenkelhalsschraube nach einem der Ansprüche 1 bis 7, **dadurch gekennzeichnet, daß** die Enden der Klingenschenkel (36, 38) außen eine Anschrägung (44) aufweisen.

9. Schenkelhalsschraube nach einem der Ansprüche 1 bis 8, **dadurch gekennzeichnet, daß** die Enden der Klingenschenkel (36, 38) innen eine Anschrägung (46) aufweisen.

10. Schenkelhalsschraube nach einem der Ansprüche 1 bis 9, **dadurch gekennzeichnet, daß** die Wände der Nuten (22, 24) im Eintrittsbereich eine Anschrägung (26) aufweisen.

11. Schenkelhalsschraube nach einem der Ansprüche 1 bis 10, **dadurch gekennzeichnet, daß** der Boden der Nuten (22, 24) im Einführbereich eine Anschrägung (28) aufweist.

## Claims

1. A neck screw (10) having a shank (12), a male-threaded portion (14) at the proximal end and surfaces for engagement by tools at the distal end wherein the screw is configured in such a way that it may be passed through a bore of a supporting device adapted to be mounted on the femur, preferably a locking nail, and to be held therein, **characterized in that** axially parallel grooves (22, 24) extending from the distal end are formed at opposite sides, o which at least one extends into said threaded portion (14), and that a fork-like blade (34) is provided the blade legs (36, 38) of which are accomodated by said grooves (22, 24) whereas the connecting portion (37) of legs (36, 38) is adapted to be connected to the distal end of the screw shank (12).

2. The neck screw according to claim 1, **characterized in that** said two grooves (22, 24) and said blade legs (36, 38) extend into said threaded portion (14).

3. The neck screw according to claim 2, **characterized in that** said blade legs (36, 38) extend beyond the proximal end of screw (10).

4. The neck screw according to any of claims 1 to 3, **characterized in that** said grooves (22, 24) and said blade legs (36, 38) are dimensioned in the area of the shank (12) in such a way that the outer surfaces of blade legs (36, 38) are located approximately at the level of the outer surface of shank (12).

5. The neck screw according to claim 4, **characterized in that** said grooves (22, 24) are more planar in the area of said threaded portion (14) and have a ramp portion between the groove portions of different depth, if required.

6. The neck screw according to any of claims 1 to 5, **characterized in that** said blade legs (36, 38) are integral with an annularly cylindrical portion (37) the outer diameter of which is approximately equal to the outer diameter of said screw shank (12), and that a screw (50) is provided for fastening the annularly cylindrical portion (37) to the distal end of said screw shank (12).

7. The neck screw according to claim 6, **characterized in that** said annularly cylindrical portion (37) has a female thread (40) for mounting to a drive-in instrument.

8. The neck screw according to any of claims 1 to 7, **characterized in that** the ends of said blade legs (36, 38) have a chamfer (44) at the outside.

9. The neck screw according to any of claims 1 to 8, **characterized in that** the ends of said blade legs (36, 38) have a chamfer (46) at the inside.

10. The neck screw according to any of claims 1 to 9, **characterized in that** the walls of said grooves (22, 24) have a chamfer (26) in the entry area.

11. The neck screw according to any of claims 1 to 10, **characterized in that** the bottom of said grooves (22, 24) have a chamfer (28) in the insertion area.

## Revendications

1. Vis pour col du fémur (10), avec une tige (12), une partie filetée extérieurement (14) à l'extrémité proximale et des surfaces de prise d'outil à l'extrémité distale, la vis ayant une configuration telle qu'elle peut être introduite à travers un perçage d'un dispositif de renfort apte à être fixé sur le fémur, de préférence une broche de verrouillage, et maintenue dans celui-ci, **caractérisée en ce que** des rainures (22, 24) sont formées parallèlement à l'axe à partir de l'extrémité distale sur des faces opposées, dont l'une au moins s'étend jusque dans la partie filetée (14) et **en ce qu'**il est prévu une lame en forme de fourche (34) dont les branches de lame (36, 38) se logent dans les rainures (22, 24) tandis que le segment de liaison (37) des branches (36, 38) peut être relié à l'extrémité distale de la tige de vis (12).

2. Vis pour col du fémur selon la revendication 1, **caractérisée en ce que** les deux rainures (22, 24) et les branches de lame (36, 38) s'étendent jusque dans la partie filetée (14).

3. Vis pour col du fémur selon la revendication 2, **caractérisée en ce que** les branches de lame (36, 38) s'étendent au-delà de l'extrémité proximale de la vis (10).

4. Vis pour col du fémur selon l'une des revendications 1 à 3, **caractérisée en ce que** les rainures (22, 24) ou les branches de lame (36, 38) sont dimensionnées dans la zone de la tige (12) de telle façon que les faces extérieures des branches de lame (36, 38) se situent approximativement à la hauteur de la face extérieure de la tige (12).

5. Vis pour col du fémur selon la revendication 4, **caractérisée en ce que** les rainures (22, 24) sont plus plates dans la zone de la partie filetée (14) et, le cas échéant, présentent un segment de rampe entre les segments de rainures de profondeur différente.

6. Vis pour col du fémur selon l'une des revendications 1 à 5, **caractérisée en ce que** les branches de lame (36, 38) sont formées d'une seule pièce avec un segment en forme de bague cylindrique (37) dont le diamètre extérieur est approximativement égal au diamètre extérieur de la tige de vis (12) et **en ce qu'**une vis (50) est prévue pour fixer le segment en forme de bague cylindrique (37) à l'extrémité distale de la tige de vis (12).

7. Vis pour col du fémur selon la revendication 6, **caractérisée en ce que** le segment en forme de bague cylindrique (37) présente un filetage intérieur (40) pour le montage sur un instrument d'impact.

8. Vis pour col du fémur selon l'une des revendications 1 à 7, **caractérisée en ce que** les extrémités des branches de lame (36, 38) présentent à l'extérieur un chanfreinage (44).

9. Vis pour col du fémur selon l'une des revendications 1 à 8, **caractérisée en ce que** les extrémités des branches de lame (36, 38) présentent à l'intérieur un chanfreinage (46).

10. Vis pour col du fémur selon l'une des revendications 1 à 9, **caractérisée en ce que** les parois des rainures (22, 24) présentent un chanfreinage (26) dans la zone d'entrée.

11. Vis pour col du fémur selon l'une des revendications 1 à 10, **caractérisée en ce que** le fond des rainures (22, 24) présente un chanfreinage (28) dans la zone d'introduction.
